(19) Europäisches Patentamt
European Patent Office
Office européen des brevets

(11) **EP 4 180 461 A1**

(12) **EUROPEAN PATENT APPLICATION**
published in accordance with Art. 153(4) EPC

(43) Date of publication:
**17.05.2023 Bulletin 2023/20**

(21) Application number: **21838008.7**

(22) Date of filing: **09.07.2021**

(51) International Patent Classification (IPC):
***C08B 15/04*** *(2006.01)*

(52) Cooperative Patent Classification (CPC):
**C08B 15/04**

(86) International application number:
**PCT/JP2021/025937**

(87) International publication number:
**WO 2022/009980 (13.01.2022 Gazette 2022/02)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.07.2020 JP 2020118642**

(71) Applicant: **Toagosei Co., Ltd.**
**Tokyo, 105-0003 (JP)**

(72) Inventors:
• **MATSUKI, Shiroshi**
  **Nagoya-shi, Aichi 455-0026 (JP)**
• **TSUIKI, Toshihiko**
  **Nagoya-shi, Aichi 455-0026 (JP)**
• **TAKADA, Jun**
  **Nagoya-shi, Aichi 455-0026 (JP)**
• **KAYANO, Hidenari**
  **Nagoya-shi, Aichi 455-0026 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **NANOCELLULOSE AND DISPERSION THEREOF**

(57) The present invention provides a nanocellulose that does not contain a N-oxyl compound in the cellulose fibers, exhibits an excellent dispersion stability in dispersion media, is the oxide of a cellulose raw material by a hypochlorous acid or a salt thereof, and has an average fiber width of from at least 1 nm to not more than 200 nm. The nanocellulose substantially does not contain a N-oxyl compound and has a zeta potential of less than or equal to -30 mV.

EP 4 180 461 A1

## Description

## Technical Field

**The** present invention relates to a nanocellulose and dispersions thereof. More particularly, the present invention relates to a nanocellulose obtained by the fibrillation treatment of an oxidized cellulose provided by the oxidation of a cellulose raw material by an oxidant, and relates to nanocellulose dispersions that contain this nanocellulose.

## Background Art

Various art has been proposed with regard to producing nanocellulose materials, e.g., cellulose nanofiber (also referred to as CNF in the following), by the oxidation of various types of cellulose raw materials with an oxidant and the microfine-sizing of the resulting oxidized cellulose (refer, for example, to Patent Document 1 and Patent Document 2).
Patent Document 1 discloses the fibrillation and nanosizing of an oxidized cellulose obtained by the oxidation of a cellulose raw material using a hypochlorous acid or a salt thereof as the oxidant at high concentration conditions of a 14 to 43 mass% available chlorine concentration in the reaction system. Patent Document 2 discloses the fibrillation and nanosizing of an oxidized cellulose obtained by the oxidation of a cellulose raw material while adjusting the pH to 5.0 to 14.0 and using a hypochlorous acid or a salt thereof as the oxidant and using an available chlorine concentration in the reaction system of 6 to 14 mass%. In this art, the oxidation process is carried out without using an N-oxyl compound, e.g., 2,2,6,6-tetramethyl-1-piperidine-N-oxy radical (TEMPO), as the catalyst, and as a consequence an N-oxyl compound does not present in the cellulose fiber and it thus becomes possible to produce a nanocellulose material while devising a reduction in the effects on, e.g., the environment.

Citation List

Patent Documents

Patent Document 1: WO 2018/230354

Patent Document 2: WO 2020/027307

## Summary

Technical Problem

In order to facilitate mixing of a nanocellulose material with other materials (for example, resins), nanocellulose materials are used in a state of dispersion in a dispersion medium, e.g., water and/or organic solvent, and so forth. In addition, nanocellulose materials are also used in the form of slurries provided by mixing with a dispersion medium and inorganic particles, e.g., a pigment. In these cases, the nanocellulose material must exhibit an excellent dispersion stability in the dispersion medium.
The present invention was pursued in view of these circumstances, and the main object of the present invention is to provide a nanocellulose that does not contain N-oxyl compounds in the cellulose fibers and that exhibits an excellent dispersion stability in dispersion media.

Solution to Problem

The present invention provides the following means in order to solve the aforementioned problem.

[1] A nanocellulose that is an oxide of a cellulose raw material by a hypochlorous acid or a salt thereof and that has an average fiber width of from at least 1 nm to not more than 200 nm, wherein the nanocellulose substantially does not comprise a N-oxyl compound and has a zeta potential of less than or equal to -30 mV.

[2] The nanocellulose of [1], wherein the average fiber width is from at least 1 nm to not more than 5 nm.

[3] The nanocellulose of [1] or [2], wherein the nanocellulose has an aspect ratio of from at least 20 to not more than 150.

[4] The nanocellulose of any of [1] to [3], wherein a mixture made by mixing the nanocellulose with water to give a solids concentration of 0.1 mass% has a light transmittance of at least 95%.

[5] A nanocellulose that is an oxide of a cellulose raw material by a hypochlorous acid or a salt thereof, wherein the nanocellulose does not comprise a N-oxyl compound and has an average fiber width of from at least 1 nm to not more than 5 nm.

[6] A nanocellulose that is an oxide of a cellulose raw material by a hypochlorous acid or a salt thereof and that has an average fiber width of from 1 nm to not more than 200 nm, wherein the nanocellulose does not comprise a N-oxyl compound and has an aspect ratio of from at least 20 to not more than 150.

[7] A nanocellulose that is an oxide of a cellulose raw material by a hypochlorous acid or a salt thereof and that has an average fiber width of from at least 1 nm to not more than 200 nm, wherein the nanocellulose does not comprise a N-oxyl compound and has a light transmittance of at least 95% for a mixture thereof made by mixing with water to give a solids concentration of 0.1 mass%.

[8] A nanocellulose dispersion in which the nanocellulose of any of [1] to [7] is dispersed in a dispersion medium.

Advantageous Effects of Invention

A nanocellulose having an excellent dispersion stability in dispersion media can be obtained in accordance with the present invention. In addition, the impacts on, inter alia, the environment can be reduced due to the absence of N-oxyl compounds.

**Description of Embodiments**

<< The nanocellulose >>

The nanocellulose according to the present disclosure (also referred to hereafter as the "present nanocellulose") is a fibrous nanocellulose provided by the fibrillation of an oxidized cellulose that is itself provided by the oxidation of a cellulose raw material with a hypochlorous acid or a salt thereof. This oxidized cellulose may also be referred to as an oxide of a cellulose raw material by a hypochlorous acid or a salt thereof. The present nanocellulose is particularly described in the following.
"Nanocellulose" is also referred to as "microfine cellulose fiber" because it is a fibrous cellulose provided by the microfine-sizing of oxidized fibrous cellulose.
The nanocellulose substantially does not comprise a N-oxyl compound because the cellulose raw material is oxidized with a hypochlorous acid or a salt thereof. In this Description, "substantially does not comprise a N-oxyl compound" means that either a N-oxyl compound is entirely absent from the nanocellulose or the content of a N-oxyl compound, with reference to the total amount of the nanocellulose, is not more than 2.0 mass-ppm, with not more than 1.0 mass-ppm being preferred. In addition, "substantially does not comprise a N-oxyl compound" also refers to the case in which the N-oxyl compound content, as an increment from the cellulose raw material, is preferably not more than 2.0 mass-ppm and more preferably not more than 1.0 mass-ppm.
By having the nanocellulose be substantially free of N-oxyl compounds, the residual presence of N-oxyl compounds, which are concerning with regard to their impact on the environment and the human body, in the nanocellulose can be inhibited. The N-oxyl compound content can be measured by a known means. Methods that use a total trace nitrogen analyzer are an example of a known means. Specifically, the N-oxyl compound-derived nitrogen component in the nanocellulose can be measured as the amount of nitrogen using a total trace nitrogen analyzer (for example, instrument name: TN-2100H, from Mitsubishi Chemical Analytech Co., Ltd.).

• First Embodiment

[The fiber width]

The average fiber width of the present nanocellulose is 1 to 200 nm. When the average fiber width exceeds 200 nm, the proportion of coarse or oversized nanocellulose then assumes high levels. As a consequence, when a nanocellulose dispersion is prepared by dispersing the nanocellulose in a dispersion medium, substantial sedimentation of the nanocellulose occurs and a trend of declining quality is assumed. Moreover, because the quality of the nanocellulose dispersion is nonuniform, when, for example, a slurry is prepared that additionally contains solid particles, e.g., of a pigment (such a slurry is also referred to in the following as a "nanocellulose-containing slurry"), the viscosity of the slurry will be unstable, and a trend is set up wherein reductions in the handling characteristics and coatability are facilitated. Viewed from such a perspective, the average particle diameter of the present nanocellulose is preferably not more than 50 nm, more preferably not more than 10 nm, and particularly preferably not more than 5 nm. In addition, when the average fiber width is less than 1 nm, this approaches a cellulose single molecule state and the quality as nanocellulose readily becomes nonuniform, and, when made into a slurry, a trend is set up wherein reductions in the viscosity stability, handling characteristics, and coatability are facilitated. As a consequence of these considerations, the average fiber width is preferably at least 1.2 nm and more preferably at least 1.5 nm.

In particular, it is advantageous for the average fiber width of the present nanocellulose to be 1 to 5 nm because this provides dispersion stability in dispersion media and, when the nanocellulose-containing slurry is prepared, a good viscosity stability, handling characteristics, and coatability can be obtained for the slurry.

[The aspect ratio]

The aspect ratio (average fiber length/average fiber width), which represents the ratio between the average fiber length and the average fiber width, for the present nanocellulose is preferably 20 to 150. When the aspect ratio is not greater than 150, this facilitates the formation of a uniform, fine, and intricate network of the microfine cellulose in the dispersion medium, and the dispersion stability can be improved due to the assumption of a stable structure. Moreover, when the nanocellulose-containing slurry is prepared, the formation of a uniform, fine, and intricate network of the solid particles and microfine cellulose is facilitated, aggregation of the solid particles can be inhibited, and the dispersion stability can be improved. In addition, aggregation between the solid particles and nanocellulose, or aggregation of the nanocellulose with itself, can be inhibited, and improvements in the slurry handling characteristics and a suppression of application unevenness can be devised. Viewed from these perspectives, the aspect ratio is more preferably not more than 145, still more preferably not more than 130, even more preferably not more than 120, and still more preferably not more than 100.

When, on the other hand, the aspect ratio is excessively low, i.e., when the shape of the nanocellulose is more that of a thick bar than a long and narrow fiber shape, network formation is then impeded, aggregation due to uneven distribution occurs, and a trend is assumed wherein reductions in the viscosity stability of the slurry are facilitated. In addition, because the slurry takes on a high viscosity, the handling characteristics readily decline, and the coating characteristics of the slurry may deteriorate. As a consequence of the preceding, the aspect ratio is more preferably at least 30, still more preferably at least 35, and even more preferably at least 40.

The average fiber width and average fiber length are the values calculated as follows: water is mixed with the nanocellulose so as to provide a nanocellulose concentration of about 1 to 10 ppm; the satisfactorily diluted aqueous cellulose dispersion is naturally dried on a mica substrate; observation of the shape of the nanocellulose is then carried out using a scanning probe microscope; some number of fibers are randomly selected from the resulting image; and the cross-sectional height of the shape image = fiber width and the length of the perimeter ÷ 2 = fiber length. Image processing software may be used for this calculation of the average fiber width and average fiber length. When this is done, the image processing conditions may be freely selected; however, there will be instances in which, even for the same image, differences in the calculated values may be produced depending on the image processing conditions. The range of difference in the values due to the image processing conditions is preferably in the ± 100 nm range for the average fiber length. The range of difference in the values due to the conditions is preferably in the ± 10 nm range for the average fiber width. More specifically, the measurement method follows the method described in the examples below.

The present nanocellulose advantageously has a structure in which at least two of the hydroxyl groups in the glucopyranose ring constituting cellulose have been oxidized, and more specifically has a structure in which the carboxy group has been introduced by the oxidation of the hydroxyl groups at positions 2 and 3 of the glucopyranose ring. Preferably the hydroxyl group at position 6 in the glucopyranose ring is not oxidized in the present nanocellulose and remains a hydroxyl group. The positions of the carboxy groups in the glucopyranose rings present in the nanocellulose can be analyzed using solid-state $^{13}$C-NMR spectroscopy.

The presence of an oxidized structure can be confirmed by observation in this solid-state $^{13}$C-NMR spectrum of a peak corresponding to the carboxy groups at positions 2 and 3 in the glucopyranose ring. Here, the peak corresponding to the carboxy groups at positions 2 and 3 can be observed as a broad peak in the range of 165 ppm to 185 ppm. The broad peak referenced here can be assessed using the peak area ratio.

That is, a baseline is drawn for the peak in the 165 ppm to 185 ppm range in the NMR spectrum; the overall area value is determined; the ratio of the two peak area values (larger area value/smaller area value) - obtained by the perpendicular partitioning of the area value at the peak top - is then determined; and the conclusion is drawn that this is a broad peak when this peak area value ratio is at least 1.2.

The presence/absence of this broad peak can also be evaluated using the ratio between the length L of the baseline for the range of 165 ppm to 185 ppm and the length L' of the perpendicular line from the peak top to the baseline.

Thus, it can be concluded that the broad peak is present when the ratio L'/L is greater than or equal to 0.1. This ratio L7L may be greater than or equal to 0.2, greater than or equal to 0.3, greater than or equal to 0.4, or greater than or equal to 0.5. While the upper limit for the ratio L7L is not particularly limited, in general it should be less than or equal to 3.0 and may be less than or equal to 2.0 or less than or equal to 1.0.

The structure of the aforementioned glucopyranose ring in the present nanocellulose can also be determined by analysis based on the methodology described in Sustainable Chem. Eng. 2020, 8, 48, 17800-17806.

[The zeta potential]

In a favorable embodiment of the present disclosure, the zeta potential of the present nanocellulose is less than or equal to -30 mV. When the zeta potential is less than or equal to -30 mV (i.e., the absolute value is greater than or equal to 30 mV), a satisfactory repulsion between microfibrils is then obtained and the production of nanocellulose having a high surface charge density during mechanical fibrillation is facilitated. This serves to improve the dispersion stability of the nanocellulose and, when the slurry is prepared, makes it possible to provide a slurry that exhibits an excellent viscosity stability, excellent handling characteristics, and excellent coating characteristics. There are no particular limitations on the lower limit for the zeta potential considered in terms of the dispersion stability. However, when the zeta potential is greater than or equal to -100 mV (i.e., the absolute value is less than or equal to 100 mV), a trend is assumed wherein oxidative cleavage in the fiber direction accompanying the development of oxidation is inhibited, and as a consequence a nanocellulose of uniform size can be obtained and excellent coating characteristics can be exhibited.

Based on the preceding considerations, the zeta potential of the present nanocellulose is preferably less than or equal to -35 mV, more preferably less than or equal to -40 mV, and still more preferably less than or equal to -50 mV. In addition, the lower limit on the zeta potential is preferably greater than or equal to -90 mV, more preferably greater than or equal to -85 mV, still more preferably greater than or equal to -80 mV, even more preferably greater than or equal to -77 mV, still more preferably greater than or equal to -70 mV, and still more preferably greater than or equal to -65 mV. Ranges for the zeta potential can be established by suitable combinations of these upper limits and lower limits. The zeta potential is preferably from greater than or equal to -90 mV to less than or equal to -30 mV, more preferably from greater than or equal to -85 mV to less than or equal to -30 mV, still more preferably from greater than or equal to -80 mV to less than or equal to -30 mV, even more preferably from greater than or equal to -77 mV to less than or equal to -30 mV, still more preferably from greater than or equal to -70 mV to less than or equal to -30 mV, still more preferably from greater than or equal to -65 mV to less than or equal to -30 mV, and still more preferably from greater than or equal to -65 mV to less than or equal to -35 mV. In the present Description, the zeta potential is the value measured, using conditions of a pH of 8.0 and 20°C, on the aqueous cellulose dispersion prepared by mixing the nanocellulose and water to provide a nanocellulose concentration of 0.1 mass%. The measurement can be specifically carried out according to the conditions indicated in the examples below.

[The light transmittance]

A nanocellulose dispersion of the present nanocellulose dispersed in a dispersion medium exhibits, inter alia, little light scattering by the cellulose fibers and can exhibit a high light transmittance. Specifically, in an advantageous embodiment, the present nanocellulose exhibits a light transmittance of at least 95% for the mixture provided by mixing with water to give a solids concentration of 0.1 mass%. As a consequence, the present nanocellulose, and nanocellulose dispersions containing same, are useful because they can also be broadly applied in applications where transparency is required. This light transmittance is more preferably at least 96%, still more preferably at least 97%, and even more preferably at least 99%. The light transmittance is the value measured at a wavelength of 660 nm using a spectrophotometer.

[Methods for producing the nanocellulose]

Methods for producing the present nanocellulose are described in the following. The present nanocellulose can be produced by a method comprising a step A of obtaining an oxidized cellulose by the oxidation of a cellulose raw material using a hypochlorous acid or a salt thereof, and a step B of fibrillating the oxidized cellulose.

The "oxidized cellulose" is also referred to as "oxidized cellulose fiber" because it is an oxidized fibrous cellulose.

(Step A: Production of oxidized cellulose)

The cellulose raw material should be a material that is mainly cellulose, but is not otherwise particularly limited, and can be exemplified by pulp, natural cellulose, regenerated cellulosed, and microfine celluloses provided by depolymerizing a cellulose by mechanical treatment. A commercial product, e.g., crystalline cellulose sourced from pulp, can be used as such as the cellulose raw material. Otherwise, unused biomass containing large amounts of a cellulose component, e.g., soy pulp or soybean skin, may be used as a raw material. The cellulose raw material may be pretreated with an alkali at a suitable concentration with the goal of facilitating permeation of the oxidant used into the starting pulp.

The hypochlorous acid or salt thereof used to oxidize the cellulose raw material can be exemplified by an aqueous hypochlorous acid, sodium hypochlorite, potassium hypochlorite, calcium hypochlorite, and ammonium hypochlorite. Among these, sodium hypochlorite is preferred from the standpoint of ease of handling.

Methods for producing the oxidized cellulose by the oxidation of a cellulose raw material can be exemplified by a method in which the cellulose raw material is mixed with a reaction solution that contains a hypochlorous acid or a salt thereof. The solvent in the reaction solution is preferably water from the standpoint of ease of handling and from the standpoint of impeding secondary reactions. The available chlorine concentration in the reaction solution of the hypochlorous acid or salt thereof is preferably 6 to 43 mass%, more preferably 7 to 43 mass%, still more preferably 10 to 43 mass%, and even more preferably 14 to 43 mass%. When the available chlorine concentration in the reaction solution is in the indicated range, the carboxy group content in the oxidized cellulose can then be satisfactorily high and fibrillation of the oxidized cellulose can be easily performed when nanocellulose production is carried out.

From the standpoint of efficiently providing a satisfactorily high carboxy group content in the oxidized cellulose, the available chlorine concentration in the reaction solution is more preferably at least 15 mass%, still more preferably at least 18 mass%, and even more preferably at least 20 mass%. Moreover, based on a consideration of inhibiting excessive decomposition of the cellulose during fibrillation, the available chlorine concentration of the reaction solution is more preferably not more than 40 mass% and still more preferably not more than 38 mass%. Ranges for the available chlorine concentration of the reaction solution can be established by suitable combinations of these upper limits and lower limits. The range for this available chlorine concentration is more preferably 16 to 43 mass% and still more preferably 18 to 40 mass%.

The available chlorine concentration of a hypochlorous acid or salt thereof is defined as follows. The hypochlorous acid is a weak acid occurring as the aqueous solution, and hypochlorites are compounds in which the hydrogen of the hypochlorous acid has been converted into another cation. For example, since sodium hypochlorite, which is a salt of the hypochlorous acid, exists in solvent (preferably in an aqueous solution), the concentration is measured as the available amount of chlorine in solution rather than the sodium hypochlorite concentration. With respect here to the available chlorine from sodium hypochlorite, the oxidizing power of the divalent oxygen atom generated by the decomposition of sodium hypochlorite corresponds to 2 atom-equivalents of monovalent chlorine, and thus the bound chlorine atom of sodium hypochlorite (NaClO) has the same oxidizing power as two atoms of nonbound chlorine ($Cl_2$) and the available chlorine = 2 × (chlorine in NaClO). In a specific measurement means, the available chlorine concentration is measured by precisely weighing the sample; adding water, potassium iodide, and acetic acid; allowing the mixture to stand; and titrating the liberated iodine with a sodium thiosulfate solution using an aqueous starch solution as indicator.

The oxidation reaction of the cellulose raw material by a hypochlorous acid or a salt thereof should be carried out while adjusting the pH into the range from 5.0 to 14.0. Within this range, the oxidation reaction of the cellulose raw material can be satisfactorily developed and the amount of carboxy group in the oxidized cellulose can be brought to satisfactorily high levels. This in turn makes it possible to readily carry out fibrillation of the oxidized cellulose. The pH of the reaction system is more preferably greater than or equal to 6.0, still more preferably greater than or equal to 7.0, and even more preferably greater than or equal to 8.0. The upper limit on the pH of the reaction system is more preferably less than or equal to 13.5 and still more preferably less than or equal to 13.0. The pH range for the reaction system is more preferably 7.0 to 14.0 and still more preferably 8.0 to 13.5.

The method for producing the oxidized cellulose is further described in the following for the example of the use of a hypochlorous acid or sodium hypochlorite as the salt thereof.

When oxidation of the cellulose raw material is carried out using sodium hypochlorite, the reaction solution is then preferably an aqueous sodium hypochlorite solution. The procedure for adjusting the available chlorine concentration of the aqueous sodium hypochlorite solution to the target concentration (for example, a target concentration of 6 mass% to 43 mass%) can be exemplified by the following: concentration of an aqueous sodium hypochlorite solution that has a lower available chlorine concentration than the target concentration; dilution of an aqueous sodium hypochlorite solution that has a higher available chlorine concentration than the target concentration; and dissolution of crystalline sodium hypochlorite (for example, sodium hypochlorite pentahydrate) in solvent. Among these, adjustment to the available chlorine concentration serving as the oxidant by dilution of an aqueous sodium hypochlorite solution or dissolution of crystalline sodium hypochlorite in solvent is preferred because there is then little autodecomposition (i.e., little decline in the available chlorine concentration) and because the available chlorine concentration may be easily adjusted.

There are no particular limitations on the method for mixing the cellulose raw material with the aqueous sodium hypochlorite solution, but, viewed from the perspective of ease of operation, mixing is preferably performed by adding the cellulose raw material to the aqueous sodium hypochlorite solution.

In order to efficiently develop the oxidation reaction of the cellulose raw material, the cellulose raw material + aqueous sodium hypochlorite solution mixture is preferably stirred during the oxidation reaction. The stirring method can be exemplified by the use of a magnetic stirrer, stir bar, stirring blade-equipped stirrer (ThreeOne Motor), homomixer, dispersing-type mixer, homogenizer, external circulation mixer, and so forth. Among these, and based on considerations of smoothly carrying out the oxidation reaction of the cellulose raw material and ease of adjusting the degree of polymerization of the oxidized cellulose to or below a prescribed value, a method using one or two or more selections from shear-based stirrers, e.g., homomixers, homogenizers, and so forth, stirring blade-equipped stirrers, and dispersing-type mixers is preferred, while a method using a stirring blade-equipped stirrer is particularly preferred. When a stirring blade-equipped stirrer is used, a device provided with known stirring blades, e.g., propeller blades, paddle blades, turbine blades, and so forth, can be used as the stirrer. When a stirring blade-equipped stirrer is used, stirring is preferably performed at a rotation rate of 50 to 300 rpm.

The reaction temperature in the oxidation reaction is preferably 15°C to 100°C and is more preferably 20°C to 90°C. The pH in the reaction system declines during the reaction accompanying the production of the carboxy group in the cellulose raw material due to the oxidation reaction. Due to this, and from the standpoint of bringing about an efficient development of the oxidation reaction, preferably a base (for example, sodium hydroxide) or an acid (for example, hydrochloric acid) is added to the reaction system to adjust the pH of the reaction system into the preferred range indicated above. The reaction time for the oxidation reaction can be established in accordance with the degree of development of the oxidation, but about 15 minutes to 50 hours is preferred. When the pH of the reaction system is made greater than or equal to 10, preferably the reaction temperature is set to 30°C or above and/or the reaction time is set to at least 30 minutes.

The fiber width and zeta potential of the nanocellulose can be adjusted to desired values by adjusting, e.g., the reaction time, reaction temperature, and stirring conditions for the oxidation reaction. Specifically, as the reaction time is lengthened and/or the reaction temperature is increased, oxidation at the cellulose microfibril surface in the cellulose raw material is more extensively developed, and a trend is set up whereby the average fiber width becomes smaller due to a strengthening of the repulsion between the fibrils due to electrostatic repulsion and osmotic pressure. In addition, the zeta potential can be increased by establishing at least one selection from the oxidation reaction time, oxidation reaction temperature, and oxidation stirring conditions on the side that brings about a greater development of oxidation (i.e., the side that causes an increase in the degree of oxidation) (for example, the reaction time can be lengthened).

The carboxy group content of the oxidized cellulose yielded by the oxidation reaction described in the preceding is preferably 0.30 to 2.0 mmol/g. When the carboxy group content of the oxidized cellulose is at least 0.30 mmol/g, a satisfactorily high fibrillatability can be imparted to the oxidized cellulose and a nanocellulose with a uniform fiber width can be obtained. This makes it possible to obtain a nanocellulose-containing slurry of uniform quality and to improve the viscosity stability, handling characteristics, and coating characteristics of the slurry. When, on the other hand, the carboxy group content is not more than 2.0 mmol/g, excessive decomposition of the cellulose during the fibrillation treatment can be inhibited and a nanocellulose of uniform quality, having a low ratio of particulate cellulose, can be obtained. In view of these considerations, the carboxy group content in the oxidized cellulose is more preferably at least 0.35 mmol/g, still more preferably at least 0.40 mmol/g, even more preferably at least 0.42 mmol/g, still more preferably at least 0.50 mmol/g, still more preferably in excess of 0.50 mmol/g, and still more preferably at least 0.55 mmol/g. The upper limit on the carboxy group content may be not more than 1.5 mmol/g, or may be not more than 1.2 mmol/g, or may be not more than 1.0 mmol/g, or may be not more than 0.9 mmol/g. Preferred ranges for the carboxy group content can be established by suitable combinations of these upper limits and lower limits. The carboxy group content of the present oxidized cellulose is more preferably 0.35 to 2.0 mmol/g, still more preferably 0.35 to 1.5 mmol/g, even more preferably 0.40 to 1.5 mmol/g, still more preferably 0.50 to 1.2 mmol/g, still more preferably from more than 0.50 to 1.2 mmol/g, and still more preferably 0.55 to 1.0 mmol/g.

The carboxy group content (mmol/g) in the oxidized cellulose can be calculated using the following formula from the amount (a) of sodium hydroxide consumed in the weak acid neutralization stage where the electrical conductivity undergoes a gentle change, by adding a 0.1 M aqueous hydrochloric acid solution to an aqueous solution containing the oxidized cellulose to bring the pH to 2.5 and then adding a 0.05 N aqueous sodium hydroxide solution dropwise and measuring the electrical conductivity until the pH reaches 11.

$$\text{carboxy group content} = a \text{ (mL)} \times 0.05 \, / \, \text{mass (g) of the oxidized cellulose}$$

An oxidized cellulose, comprising an oxide of the cellulose raw material by a hypochlorous acid or a salt thereof, can be obtained by subjecting the solution containing the oxidized cellulose yielded by the aforementioned reaction, to a known separation process, e.g., filtration, and as necessary to additional purification. In addition, based on considerations of improving the filtration behavior and yield in the separation process, at least a portion of the salt form ($-COO^- X^+$ : $X^+$ indicates a cation, e.g., sodium, lithium) of the carboxy groups produced by oxidation can be converted into the proton form (-COO⁻ H⁺) by the addition, prior to the separation process, e.g., filtration, of an acid to the oxidized cellulose-containing solution, for example, to bring the pH to 4.0 or below.

In the infrared absorption spectrum, the proton form presents a peak in the vicinity of 1720 $cm^{-1}$ and the salt form presents a peak in the vicinity of 1600 $cm^{-1}$, which enables these to be distinguished.

The oxidized cellulose-containing solution yielded by the aforementioned reaction may be supplied as such to the fibrillation treatment.

When the pH of the oxidized cellulose-containing solution has been brought to 4.0 or below for the separation process, in order to improve the handling characteristics when provided to the ensuing fibrillation treatment, at least a portion of the carboxy groups can be converted to the salt form (-COO⁻ X⁺ : X⁺ indicates a cation, e.g., sodium, lithium), for example, by bringing the pH to 6.0 or higher by the addition of a base. In addition, the oxidized cellulose-containing solution may be converted into an oxidized cellulose-containing composition, for example, by replacing the solvent in the former. At least a portion of the carboxy groups can also be converted into the salt form (-COO- X⁺ : X⁺ indicates a cation, e.g., sodium, lithium) in the oxidized cellulose-containing composition, for example, by establishing alkaline conditions of 10 or higher for the pH.

In order to control the properties of the oxidized cellulose, the method for producing the present oxidized cellulose may additionally comprise a step of mixing the obtained oxidized cellulose with a modifying group-bearing compound. The modifying group-bearing compound should be a compound that has a modifying group that can form an ionic bond or covalent bond with the carboxy groups or hydroxyl groups possessed by the oxidized cellulose, but is not otherwise particularly limited. Compounds having a modifying group that can form an ionic bond can be exemplified by primary amines, secondary amines, tertiary amines, quaternary ammonium compounds, and phosphonium compounds. Compounds having a modifying group that can form a covalent bond can be exemplified by alcohols, isocyanate compounds, and epoxy compounds.

In accordance with the preceding, the oxidized cellulose encompasses the embodiments represented by the salt forms, proton forms, and modified forms as provided by a modifying group. The nanocellulose obtained from the present oxidized cellulose also encompasses the embodiments represented by the salt forms, proton forms, and modified forms as provided by a modifying group.

(Step B: Fibrillation treatment)

The present nanocellulose can be obtained by carrying out fibrillation and nanosizing of the oxidized cellulose obtained as described above. The method of fibrillating the oxidized cellulose can be exemplified by methods that execute weak stirring using, for example, a magnetic stirrer, and by methods that perform a mechanical fibrillation. Fibrillation of the oxidized cellulose is preferably carried out using a mechanical treatment because this enables a satisfactory fibrillation of the oxidized cellulose to be carried out and enables a shortening of the fibrillation time to be devised. Here, nanocellulose (also referred to as "nanocellulose") is a general term for the products provided by the nanosizing of cellulose and encompasses, e.g., cellulose nanofiber, cellulose nanocrystals, and so forth.

The method for carrying out mechanical fibrillation can be exemplified by methods that use various mixers or stirring devices, such as, for example, screw mixers, paddle mixers, dispersing mixers, turbine mixers, high-speed homomixers, high-pressure homogenizers, ultrahigh-pressure homogenizers, double cylinder homogenizers, ultrasonic homogenizers, water-current counter-collision dispersers, beaters, disk refiners, conical refiners, double disk refiners, grinders, single-screw kneaders, multi-screw kneaders, planetary centrifugal mixers, and vibrating agitators. A single one of these devices may be used by itself or a combination of two or more of these devices may be used, and preferably nanocellulose production is carried out by nanosizing the oxidized cellulose by treating the oxidized cellulose in a dispersion medium.

From the standpoint of being able to efficiently produce nanocellulose in which fibrillation is more advanced, fibrillation of the oxidized cellulose can preferably use a method that employs an ultrahigh-pressure homogenizer. When a fibrillation treatment using an ultrahigh-pressure homogenizer is performed, the pressure during the fibrillation treatment is preferably at least 100 MPa, more preferably at least 120 MPa, and still more preferably at least 150 MPa. The number of times the fibrillation treatment is carried out is not particularly limited, but, from the standpoint of achieving a satisfactory development of the fibrillation, the fibrillation treatment is preferably carried out at least two times and more preferably at least three times. In addition, the aforementioned oxidized cellulose can also be satisfactorily fibrillated by mild stirring using, for example, a planetary centrifugal mixer or a vibrating mixer. A vortex mixer (touch mixer) is an example of a vibrating mixer. That is, a homogenized nanocellulose can also be obtained from the aforementioned oxidized cellulose when the fibrillation treatment is carried out using mild fibrillation conditions.

The fibrillation treatment is preferably carried out in a state in which the oxidized cellulose is mixed with a dispersion medium. This dispersion medium is not particularly limited and can be selected as appropriate in accordance with the objectives. Specific examples of the dispersion medium are water, alcohols, ethers, ketones, N,N-dimethylformamide, N,N-dimethylacetamide, and dimethyl sulfoxide. A single one of these may be used by itself as the solvent or two or more may be used in combination.

Of the dispersion media referenced above, the alcohols can be exemplified by methanol, ethanol, isopropanol, isobutanol, sec-butyl alcohol, tert-butyl alcohol, methyl cellosolve, ethylene glycol, and glycerol. The ethers can be exemplified by ethylene glycol dimethyl ether, 1,4-dioxane, and tetrahydrofuran. The ketones can be exemplified by acetone and methyl ethyl ketone.

Isolation of the oxidized cellulose, and of the nanocellulose yielded by its fibrillation, is facilitated by the use of an organic solvent as the dispersion medium in the fibrillation treatment. Since nanocellulose dispersed in organic solvent is then obtained, mixing with, e.g., resin soluble in the organic solvent or monomer that is a starting material for such a resin, is facilitated. The nanocellulose dispersion provided by dispersing nanocellulose yielded by fibrillation in a dispersion medium of water and/or organic solvent, can be used for mixing with various components, e.g., resins, rubbers, solid particles, and so forth.

The hereinabove described nanocellulose and nanocellulose dispersions containing same can be used in a variety of applications. In specific terms, for example, they may be used as a reinforcing material when mixed with various materials (e.g., resins, fibers, rubbers, and so forth) and may be used as a thickener or dispersant in various applications (e.g., foods, cosmetics, medical products, paints, inks, and so forth). The nanocellulose dispersion may also be formed into a film and used as various sheets and films. The fields of application of the present nanocellulose and nanocellulose dispersions containing same are not particularly limited, and they may be used in the production of products in various fields, e.g., automotive components, machine parts, electrical appliances, electronic devices, cosmetics, medical products, building materials, daily necessities, stationery, and so forth. Moreover, for example, the use of the nanocellulose or nanocellulose dispersion containing same as an additive to a slurry containing inorganic particles, such as a pigment, is advantageous from the standpoint of enabling improvements in the viscosity stability, handling characteristics, and coating performance of the slurry.

• Second Embodiment

In an advantageous embodiment of the present disclosure, the present nanocellulose is a nanocellulose provided by the oxidation of a cellulose raw material with a hypochlorous acid or a salt thereof (also referred to as nanocellulose that is an oxide of a cellulose raw material by a hypochlorous acid or a salt thereof) and does not contain N-oxyl compounds and has an average fiber width of from at least 1 nm to not more than 5 nm. When the average fiber width of the present nanocellulose is 1 to 5 nm, the zeta potential of the present nanocellulose, viewed from the perspectives of improving the dispersion stability of the nanocellulose and enabling improvements in the handling characteristics when made into a slurry, is preferably less than or equal to -25 mV and more preferably less than or equal to -30 mV. The descriptions in the first embodiment, supra, can be cited, inter alia, for the description of the method for producing the present nanocellulose.

• Third Embodiment

In an advantageous embodiment of the present disclosure, the present nanocellulose is a nanocellulose provided by the oxidation of a cellulose raw material with a hypochlorous acid or a salt thereof (also referred to as nanocellulose that is an oxide of a cellulose raw material by a hypochlorous acid or a salt thereof) and does not contain N-oxyl compounds and has an aspect ratio of from at least 20 to not more than 150. When the aspect ratio of the present nanocellulose is from at least 20 to not more than 150, the zeta potential of the present nanocellulose, viewed from the perspectives of improving the dispersion stability of the nanocellulose and enabling improvements in the handling characteristics when made into a slurry, is preferably less than or equal to -25 mV and more preferably less than or equal to -30 mV. The descriptions in the first embodiment, supra, can be cited for the description of, inter alia, the method of producing the present nanocellulose.

Examples

The present invention is specifically described in the following using examples, but the present invention is not limited to or by these examples. Unless specifically indicated otherwise, in the following "parts" indicates "mass parts" and "%" indicates "mass%".

(1) Production of oxidized celluloses and nanocelluloses

[Production Example 1]

To provide the cellulose raw material, a softwood pulp (NIST RM 8495, bleached kraft pulp, Sigma-Aldrich) was cut with scissors to 5 mm square and was mechanically fibrillated to a cotton-like form by treatment for 1 minute at 25,000 rpm with a "Wonder Blender WB-1" from Osaka Chemical Co., Ltd.

350 g of sodium hypochlorite pentahydrate crystals, which had an available chlorine concentration of 42 mass%, was introduced into a beaker and pure water was added and stirring was performed to obtain an aqueous sodium hypochlorite solution having an available chlorine concentration of 21 mass%. To this was added 35 mass% hydrochloric acid with stirring to provide an aqueous solution having a pH of 11.0. This aqueous sodium hypochlorite solution was heated to 30°C with a thermostatted water bath while stirring at 200 rpm using a stirrer from Shinto Scientific Co., Ltd. (ThreeOne Motor, BL600) and using a propeller stirring blade; this was followed by the addition of 50 g of the aforementioned mechanically fibrillated softwood kraft pulp (carboxy group content: 0.05 mmol/g).

After the introduction of the cellulose raw material, and while holding the temperature at 30°C on the same thermostatted water bath, an oxidation reaction was run for 20 minutes while stirring at 200 rpm using a propeller stirring blade and the aforementioned stirrer while adjusting the pH during the reaction to 11.0 by the addition of 48 mass% sodium hydroxide. After the completion of the reaction, solid-liquid separation was carried out on the product by suction filtration using a PTFE membrane filter having an aperture of 0.1 μm, and the resulting oxidized cellulose was washed with pure water. The carboxy group content measured post-washing for the residue (oxidized cellulose) was 0.37 mmol/g.

A 5% dispersion was then prepared by the addition of pure water to the oxidized cellulose, and treatment was performed for 10 passes at 200 MPa using a "Star Burst Labo HJP-25005" ultrahigh-pressure homogenizer from Sugino Machine Limited to obtain an aqueous CNF dispersion A in the form of a nanocellulose dispersion. In an ultrahigh-pressure homogenizer, fibrillation is advanced by the pass-through circulation of the aqueous oxidized cellulose dispersion at an ultrahigh-pressure fibrillation section built into the ultrahigh-pressure homogenizer. Passage of the liquid one time through this ultrahigh-pressure fibrillation section is designated as 1 pass.

In addition, the N-oxyl compound-derived nitrogen component in the oxidized cellulose was measured as the amount of nitrogen using a total trace nitrogen analyzer (instrument name: TN-2100H, from Mitsubishi Chemical Analytech Co., Ltd.); the increment from the starting pulp was calculated, giving a result of not more than 1 ppm.

The available chlorine concentration in the aqueous sodium hypochlorite solution was measured using the following method.

(Measurement of the available chlorine concentration in the aqueous sodium hypochlorite solution)

0.582 g of an aqueous solution of sodium hypochlorite pentahydrate crystals added to pure water was precisely weighed out, and 50 mL of pure water was added and 2 g of potassium iodide and 10 mL of acetic acid were added, followed immediately by tight sealing and standing for 15 minutes in a dark location. After the standing for 15 minutes, the liberated iodine was titrated with a 0.1 mol/L sodium thiosulfate solution, which resulted in a titration volume of 34.55 mL (indicator: starch reagent solution). Correction was performed by carrying out a separate blank test. Since 1 mL of the 0.1 mol/L sodium thiosulfate solution corresponded to 3.545 mg Cl, the available chlorine concentration in the aqueous sodium hypochlorite solution was 21 mass%.

The carboxy group content in the oxidized cellulose was measured using the following method.

(Measurement of the carboxy group content)

To 60 mL of an aqueous oxidized cellulose dispersion adjusted to an oxidized cellulose concentration of 0.5 mass%, was added 0.1 M aqueous hydrochloric acid solution to bring the pH to 2.5. This was followed by the dropwise addition of a 0.05 N aqueous sodium hydroxide solution and measurement of the electrical conductivity until the pH reached 11.0, and the carboxy group content (mmol/g) was calculated using the following formula from the amount (a) of sodium hydroxide consumed in the weak acid neutralization stage where the electrical conductivity undergoes a gentle change.

$$\text{carboxy group content} = a \text{ (mL)} \times 0.05/ \text{ mass (g) of the oxidized cellulose}$$

[Production Example 2]

An aqueous CNF dispersion B were obtained by carrying out treatment using the same conditions as in Production Example 1, but using 30 minutes for the reaction time in the oxidation reaction.

[Production Example 3]

An aqueous CNF dispersion C was obtained by carrying out treatment using the same conditions as in Production Example 1, but using 120 minutes for the reaction time in the oxidation reaction.

[Production Example 4]

An aqueous CNF dispersion D was obtained by carrying out treatment using the same conditions as in Production Example 1, but using 360 minutes for the reaction time in the oxidation reaction.

[Production Example 5]

An aqueous CNF dispersion E was obtained by carrying out treatment using the same conditions as in Production Example 1, but using 480 minutes for the reaction time in the oxidation reaction.

[Production Example 6]

An aqueous CNF dispersion F was obtained by carrying out treatment using the same conditions as in Production Example 1, but changing the reaction temperature in the oxidation reaction to 40°C from 30°C and using 120 minutes for the reaction time in the oxidation reaction.

[Production Example 7]

An aqueous CNF dispersion G was obtained by carrying out treatment using the same conditions as in Production Example 1, but changing the reaction temperature in the oxidation reaction to 50°C from 30°C and using 120 minutes for the reaction time in the oxidation reaction.

[Production Example 8]

An aqueous CNF dispersion H was obtained by carrying out treatment using the same conditions as in Production Example 1, but changing the reaction temperature in the oxidation reaction to 40°C from 30°C and using 480 minutes for the reaction time in the oxidation reaction.

[Production Example 9]

An aqueous CNF dispersion I was obtained by carrying out treatment using the same conditions as in Production Example 1, but changing the reaction temperature in the oxidation reaction to 20°C from 30°C and using 120 minutes for the reaction time in the oxidation reaction.

[Production Example 10]

An aqueous CNF dispersion J was obtained by carrying out treatment using the same conditions as in Production Example 1, but changing the reaction time in the oxidation reaction to 15 minutes.

[Production Example 11]

An aqueous CNF dispersion K was obtained by carrying out treatment using the same conditions as in Production Example 1, but changing the reaction temperature in the oxidation reaction to 15°C from 30°C and using 120 minutes for the reaction time in the oxidation reaction.
A sample was prepared by freeze-frying the oxidized cellulose provided by the particular production example and then holding for at least 24 hours at 23°C and 50% RH, and the solid-state $^{13}$C-NMR of the sample was measured. As a result, in all instances it was confirmed that a structure was present in which the hydroxyl groups at positions 2 and 3 of the glucopyranose ring were oxidized with the introduction of carboxy groups. The solid-state $^{13}$C-NMR measurement conditions are given below.

(1) sample tube: zirconia tube (4 mm diameter)

(2) magnetic field strength: 9.4 T (1H resonance frequency: 400 MHz)

(3) MAS rotation rate: 15 kHz

(4) pulse sequence: CPMAS method

(5) contact time: 3 ms

(6) waiting time: 5 s

(7) number of scans: 10,000 to 15,000

(8) measurement instrument: JNM ECA-400 (JEOL Ltd.)

The results obtained using a model molecule for said oxidized cellulose as the sample and carrying out measurement using two-dimensional NMR also confirmed that the oxidized cellulose yielded by each production example had a structure in which the hydroxyl groups at positions 2 and 3 of the glucopyranose ring were oxidized with the introduction of carboxy groups.

With regard to position 6, no change in the spectroscopic data was seen between the solid-state $^{13}$C-NMR of the cellulose raw material and the solid-state $^{13}$C-NMR of the oxidized cellulose, and based on this it was concluded that the hydroxyl group at position 6 was not oxidized and the hydroxyl group remained intact in the oxidized cellulose.

[Comparative Production Example 1]

To provide the cellulose raw material, a softwood pulp (NIST RM 8495, bleached kraft pulp, Sigma-Aldrich) was cut with scissors to 5 mm square and was mechanically fibrillated to a cotton-like form by treatment for 1 minute at 25,000 rpm with a "Wonder Blender WB-1" from Osaka Chemical Co., Ltd.

30.0 g of sodium hypochlorite pentahydrate crystals, which had an available chlorine concentration of 43 mass%, was introduced into a 100-mL beaker and pure water and 35 mass% hydrochloric acid were added with stirring to obtain an aqueous solution having an available chlorine concentration of 21 mass% and a pH of 11.0. This aqueous sodium hypochlorite solution was heated to 30°C with a thermostatted water bath while stirring with a stirrer; this was followed by the addition of 0.35 g of the aforementioned mechanically fibrillated softwood kraft pulp.

After the introduction of the cellulose raw material, and while holding the temperature at 30°C on the same thermostatted water bath, stirring with a stirrer was carried out for 30 minutes while adding 48 mass% sodium hydroxide in order to maintain pH 11.0. Solid-liquid separation was then carried out on the product by suction filtration using a PTFE membrane filter having an aperture of 0.1 $\mu$m, and the resulting residue was washed with pure water. The carboxy group content measured post-washing for the residue (oxidized cellulose) was 0.42 mmol/g.

A 5% dispersion was prepared by dispersing the obtained oxidized cellulose in pure water, and an aqueous CNF dispersion P was obtained by carrying out a fibrillation treatment for 10 minutes using a "UP-400S" ultrasound homogenizer from Hielscher Ultrasonics GmbH and using conditions of CYCLE = 0.5 and AMPLIYUDE = 50. With an ultrasound homogenizer, an ultrasound generator element is immersed in the aqueous oxidized cellulose dispersion introduced into a container, and fibrillation is developed with ultrasound generated from the ultrasound generator element.

[Comparative Production Example 2]

To provide the cellulose raw material, a softwood pulp (NIST RM 8495, bleached kraft pulp, Sigma-Aldrich) was cut with scissors to 5 mm square and was mechanically fibrillated to a cotton-like form by treatment for 1 minute at 25,000 rpm with a "Wonder Blender WB-1" from Osaka Chemical Co., Ltd.

30.3 g of sodium hypochlorite pentahydrate crystals, which had an available chlorine concentration of 42 mass%, was introduced into a beaker and pure water was added with stirring to bring the available chlorine concentration to 14 mass%. To this was added 35 mass% hydrochloric acid with stirring to obtain an aqueous solution having a pH of 9.0. This aqueous sodium hypochlorite solution was heated to 30°C with a thermostatted water bath while stirring with a stirrer; this was followed by the addition of 0.35 g of the aforementioned mechanically fibrillated softwood kraft pulp.

After the introduction of the cellulose raw material, and while holding the temperature at 30°C on the same thermostatted water bath, an oxidation reaction was run by stirring with a stirrer for 30 minutes while adding 48 mass% sodium hydroxide in order to adjust the pH during the reaction to 9.0. After the completion of the reaction, solid-liquid separation was carried out on the product by suction filtration using a PTFE mesh filter having an aperture of 0.1 $\mu$m, and the resulting residue was washed with pure water. The carboxy group content measured post-washing for the residue (oxidized cellulose) was 1.12 mmol/g.

A 5% dispersion was then prepared by the addition of pure water to the oxidized cellulose, and an aqueous CNF dispersion Q was obtained by carrying out a fibrillation treatment with an ultrasound homogenizer using the same conditions as in Comparative Production Example 1.

[Comparative Production Example 3]

To provide the cellulose raw material, a softwood pulp (NIST RM 8495, bleached kraft pulp, Sigma-Aldrich) was cut with scissors to 5 mm square and was mechanically fibrillated to a cotton-like form by treatment for 1 minute at 25,000 rpm with a "Wonder Blender WB-1" from Osaka Chemical Co., Ltd. A wet powder was obtained by thoroughly dispersing the mechanically fibrillated cellulose fibers in water and carrying out suction filtration using a PTFE mesh filter having an aperture of 0.1 $\mu$m.

This wet powder (80 mass% moisture fraction, 20 g as the dry powder) was introduced into a container, and 60 L of an ozone · oxygen mixed gas with an ozone concentration of 200 g/m$^3$ was then added and shaking was performed for 2 minutes at 25°C. After standing at quiescence for 6 hours, the ozone and so forth in the container was removed, after which the oxidized cellulose was taken out and washed with pure water by suction filtration using a PTFE mesh filter having an aperture of 0.1 $\mu$m. Pure water was added to the obtained oxidized cellulose to prepare a 2 mass% dispersion, and sodium hydroxide was added to give a 0.3 mass% sodium hydroxide solution. Stirring was carried out for 5 minutes followed by standing at quiescence for 30 minutes at 25°C. Washing with pure water was then performed by suction filtration using a PTFE mesh filter having an aperture of 0.1 $\mu$m. The carboxy group content measured post-washing for the oxidized cellulose was 0.43 mmol/g.

A 5% dispersion was prepared by the addition of pure water to this oxidized cellulose, and treatment was then performed using conditions of 10 passes at 200 MPa using a "Star Burst Labo HJP-25005" ultrahigh-pressure homogenizer from Sugino Machine Limited to obtain an aqueous CNF dispersion R.

[Comparative Production Example 4]

To provide the cellulose raw material, a softwood pulp (NIST RM 8495, bleached kraft pulp, Sigma-Aldrich) was cut with scissors to 5 mm square and was mechanically fibrillated to a cotton-like form by treatment for 1 minute at 25,000 rpm with a "Wonder Blender WB-1" from Osaka Chemical Co., Ltd.

4.92 g sodium periodate was introduced into a beaker and pure water was added to make an aqueous solution (total amount = 600 mL). This aqueous sodium periodate solution was heated to 55°C with a thermostatted water bath while stirring at 200 rpm using a stirrer from Shinto Scientific Co., Ltd. (ThreeOne Motor, BL600) and using a propeller stirring blade; this was followed by the addition of 6 g of the aforementioned mechanically fibrillated softwood kraft pulp.

After the introduction of the cellulose raw material, and while holding the temperature at 55°C on the same thermostatted water bath, stirring was carried out for 3 hours under the same conditions using a stirrer. After the completion of the reaction, the product was subjected to solid-liquid separation by suction filtration using a PTFE membrane filter having an aperture of 0.1 $\mu$m, and washing with pure water was performed.

The product obtained as described above was then added to a 1 M aqueous acetic acid solution that contained sodium chlorite, and stirring was carried out under the same stirring conditions as above for 48 hours at 25°C. After the completion of the reaction, the product was subjected to solid-liquid separation by suction filtration using a PTFE membrane filter having an aperture of 0.1 $\mu$m, and washing with pure water was performed. The carboxy group content measured post-washing for the oxidized cellulose was 1.72 mmol/g.

A 5% dispersion was prepared by the addition of pure water to the obtained oxidized cellulose, the pH was adjusted to 7.5 by the addition of an aqueous sodium hydroxide solution, and water washing was performed. The obtained dispersion was treated using conditions of 10 passes at 200 MPa using a "Star Burst Labo HJP-25005" ultrahigh-pressure homogenizer from Sugino Machine Limited to obtain an aqueous CNF dispersion S.

[Comparative Production Example 5]

To provide the cellulose raw material, a softwood pulp (NIST RM 8495, bleached kraft pulp, Sigma-Aldrich) was cut with scissors to 5 mm square and was mechanically fibrillated to a cotton-like form by treatment for 1 minute at 25,000 rpm with a "Wonder Blender WB-1" from Osaka Chemical Co., Ltd.

0.016 g TEMPO and 0.1 g sodium bromide were introduced into a beaker; pure water was added with stirring to prepare an aqueous solution; and 1.0 g of the aforementioned mechanically fibrillated softwood pulp was added.

This aqueous solution was heated to 25°C on a thermostatted water bath while stirring with a stirrer, after which 0.1 M sodium hydroxide was added with stirring to prepare an aqueous solution having a pH of 10.0. To this was added 2.58 g of an aqueous sodium hypochlorite solution having an available chlorine concentration of 13.2 mass%, and, while holding the temperature at 25°C on the same thermostatted water bath, stirring with a stirrer was performed for 120 minutes while adjusting the pH during the reaction to 10.0 by adding 0.1 M sodium hydroxide.

After the completion of the reaction, solid-liquid separation was carried out on the product by suction filtration using a PTFE membrane filter having an aperture of 0.1 $\mu$m, and the resulting residue was washed with pure water. The carboxy group content measured post-washing for the residue (oxidized cellulose) was 1.55 mmol/g. A 5% dispersion was prepared by the addition of pure water to the obtained residue, the pH was adjusted to 7.5 by the addition of an aqueous sodium hydroxide solution, and water washing was performed. The obtained dispersion was treated using conditions of 10 passes at 200 MPa using a "Star Burst Labo HJP-25005" ultrahigh-pressure homogenizer from Sugino Machine Limited to obtain an aqueous CNF dispersion T. The N-oxyl compound-derived nitrogen component in the oxidized cellulose was measured as the amount of nitrogen using the same conditions as in Production Example 1, and the result, calculated as the increment from the starting pulp, was 5 ppm.

[First Example]

[Examples 1-1 to 1-9 and Comparative Examples 1-1 to 1-5] : Examination of the zeta potential

Using the aqueous CNF dispersions prepared in each of Production Examples 1 to 9 and Comparative Production Examples 1 to 5, the following evaluations were carried out and the relationship between the zeta potential of the nanocellulose and its dispersion stability was examined. The results of the evaluations are given in Table 1.

[Measurement of the zeta potential]

Dilution to a nanocellulose concentration of 0.1% was performed by adding pure water to each of the aqueous CNF dispersions A to I and P to T obtained as described in the preceding. After dilution, a 0.05 mol/L aqueous sodium hydroxide solution was added to the particular aqueous CNF dispersion to adjust the pH to 8.0, and the zeta potential was then measured at 20°C using a zeta potential analyzer (ELSZ-1000) from Otsuka Electronics Co., Ltd.

[Measurement of average fiber width]

Pure water was added to each of the aqueous CNF dispersions A to I and P to T obtained as described above in order to adjust the nanocellulose concentration in the aqueous CNF dispersion to 5 ppm. After the concentration adjustment, the aqueous CNF dispersion was naturally dried on a mica substrate, and shape observation of the nanocellulose was performed in AC mode using an "MFP-3D Infinity" from Oxford · Asylum. For the average fiber width, using the software provided with the "MFP-3D Infinity", the number-average fiber width [nm] was determined using cross-sectional height in shape image = fiber width for at least 50 of the fibers.

[Measurement of the light transmittance]

Each of the aqueous CNF dispersions A to I and P to T obtained as described above was introduced into a 10 mm-thick quartz cell, and the light transmittance at a wavelength of 660 nm was measured using a spectrophotometer (JASCO V-550).

[Stability of the aqueous CNF dispersions]

Pure water was added to each of the aqueous CNF dispersions A to I and P to T to dilute same to a nanocellulose concentration in the aqueous CNF dispersion of 0.1 mass%, and storage was carried out by holding at quiescence for 4 weeks at 25°C. The solids concentration in the supernatant was measured both immediately after dilution and after the 4-week storage period. The dispersion ratio was calculated using the following formula, and the dispersion stability was evaluated using the following evaluation criteria. The solids concentration was calculated using the mass change pre-versus-post-execution of a drying treatment at 105°C.

dispersion ratio (%) = (solids concentration after 4 weeks/solids concentration immediately after dilution) × 100

+ + : the dispersion ratio is at least 95%
+ : the dispersion ratio is at least 90%, but less than 95%
Δ : the dispersion ratio is at least 85%, but less than 90%
✕ : the dispersion ratio is less than 85%

[Slurry viscosity stability]

Water-based slurries (50 g) containing 5 mass% titanium oxide (R-820, Ishihara Sangyo Kaisha, Ltd.) and each particular aqueous CNF dispersion A-I, P-T were prepared, while changing the amount of nanocellulose addition so the initial viscosity, i.e., the viscosity immediately after preparation of the slurry, was the same in each example (300 mPa · s). A "THINKYMIXER ARE-310" mixer from the THINKY Corporation (mix mode, revolution rate = 2,000 rpm, rotation rate = 800 rpm, 20 minutes) was used for the mixing to prepare the water-based slurries.
The viscosity was measured immediately after preparation (initial viscosity) and after standing at quiescence for 1 week, and the percentage change in the viscosity was calculated using the following formula and the viscosity stability of the water-based slurry was evaluated using the evaluation criteria provided below.

$$\text{percentage change in viscosity (\%)} = (N2/N1) \times 100$$

(In the formula, N1 is the initial viscosity of the slurry and N2 is the viscosity of the slurry after standing at quiescence for 1 week after sample preparation.)

+ + : the percentage change in viscosity is less than 105%

+ : the percentage change in viscosity is at least 105% but less than 110%

Δ : the percentage change in viscosity is at least 110% but less than 115%

× : the percentage change in viscosity is at least 115%

Standing at quiescence was carried out in a room (23 ± 2°C).
The initial viscosity of the slurry and its viscosity after standing at quiescence for 1 week were measured using the following conditions: after stirring with a spatula at a speed at which bubbles were not introduced, the measurement was performed using an E-type viscometer (TV-22) from Toki Sangyo Co., Ltd. at 25°C and 100 rpm (shear rate = 200 s$^{-1}$).

[Slurry handling characteristics]

A process liquid was prepared by adding water to each of the aqueous CNF dispersions A to I and P to T to provide 5 mass% of the aluminum silicate powder and 0.5 mass% nanocellulose, with blending and stirring. This process liquid was lightly stirred with a spatula and then scooped up, and dripping of the liquid when the spatula was tilted was visually observed and the slurry handling characteristics were evaluated using the following criteria.

+ + : Fluid dripping was produced immediately upon tilting.

+ : Fluid dripping was produced from 5 seconds after tilting.

Δ : Fluid dripping was produced from 10 seconds after tilting.

× : Fluid dripping was not produced even after 15 seconds.

[Surface condition after coating with slurry (coating characteristics)]

A process liquid was prepared by adding water to each of the aqueous CNF dispersions A to I and P to T to provide 5 mass% of the aluminum silicate powder and 0.5 mass% nanocellulose, with blending and stirring. The process liquid was coated on a woven fabric (100% polyester, 100 mm × 100 mm) to provide an aluminum silicate powder application amount of 5 g/m$^2$, and drying was carried out. 10 sheets of the coated woven fabric were visually inspected for nonuniform locations of application (application unevenness), and evaluation was performed using the following criteria.

+ + : Application unevenness was not seen on any of the 10 sheets.

+ : Application unevenness was not seen on 8-9 sheets.

Δ      : Application unevenness was not seen on 4 to 7 sheets.

×      : Application unevenness was not seen on 1 to 3 sheets, or application unevenness was seen on all 10 sheets.

[Table 1]

| | designation of aqueous dispersion | N-oxyl compound | oxidation method | carboxy group content (mmol/g) | zeta potential (mV) | average fiber width (nm) | light transmittance (%) | stability of the aqueous CNF dispersion | | slurry viscosity stability | slurry handling characteristics | surface condition after coating with slurry |
|---|---|---|---|---|---|---|---|---|---|---|---|---|
| | | | | | | | | dispersion ratio (%) | rank | | | |
| Example 1-1 | A | × | hypochlorite | 0.37 | -30.2 | 4.7 | 95.5 | 94.2 | + | + | + | + |
| Example 1-2 | B | | | 0.45 | -32.5 | 4.5 | 97.8 | 98.9 | + + | + | + | + |
| Example 1-3 | C | | | 0.61 | -52.9 | 3.7 | 99.2 | 99.4 | + + | + + | + + | + + |
| Example 1-4 | D | | | 0.92 | -65.7 | 2.7 | 99.5 | 99.5 | + + | + + | + + | + + |
| Example 1-5 | E | | | 0.97 | -75.6 | 2.3 | 99.7 | 99.7 | + + | + | + + | + |
| Example 1-6 | F | | | 0.94 | -67.8 | 2.3 | 99.5 | 99.4 | + + | + + | + + | + + |
| Example 1-7 | G | | | 0.95 | -70.2 | 2.1 | 99.6 | 99.5 | + + | + | + + | + |
| Example 1-8 | H | | | 1.15 | -80.5 | 2.0 | 99.8 | 99.8 | + + | Δ | + + | Δ |
| Example 1-9 | I | | | 0.39 | -31.9 | 5.3 | 96.5 | 95.7 | + + | Δ | + | Δ |
| Comparative Example 1-1 | P | × | hypochlorite | 0.42 | -17.9 | 5.3 | 84.7 | 83.3 | × | Δ | Δ | Δ |
| Comparative Example 1-2 | Q | | | 1.12 | -21.7 | 5.2 | 90.7 | 84.7 | × | Δ | Δ | Δ |
| Comparative Example 1-3 | R | | ozone oxidation | 0.43 | -33.6 | 5.5 | 78.6 | 83.8 | × | Δ | Δ | Δ |
| Comparative Example 1-4 | S | | periodic acid | 1.72 | -16.0 | 19.3 | 78.0 | 80.5 | × | × | × | × |
| Comparative Example 1-5 | T | + | TEMPO oxidation | 1.55 | -70.9 | 3.4 | 99.4 | 99.5 | + + | × | × | × |

In Table 1, an "×" is used to indicate the case in which an N-oxyl compound was not used during the oxidation treatment of the cellulose raw material (i.e., the CNF dispersion substantially did not contain N-oxyl compounds), and a "+" is used to indicate the case in which an N-oxyl compound was used (i.e., an N-oxyl compound was present in the CNF dispersion) (the same applies to Table 2 and 3 below).

As shown in Table 1, when Comparative Examples 1-1 and 1-2 are compared with Examples 1-1 to 1-9, in which examples nanocellulose was produced by an oxidation treatment using a hypochlorite, Examples 1-1 to 1-9, in which the zeta potential was less than or equal to -30 mV, gave better slurry characteristics than in Comparative Examples 1-1 and 1-2, in which the zeta potential was -17.9 mV and -21.7 mV, respectively.

Specifically, the dispersion stability of the nanocellulose was high in the aqueous CNF dispersions of Examples 1-1 to 1-9. In addition, a balance was achieved among the viscosity stability, handling characteristics, and coating characteristics for the slurries obtained in Examples 1-1 to 1-9. In particular, in Examples 1-1 to 1-7, the viscosity stability, handling characteristics, and coating characteristics of the slurries were all evaluated with a "+ +" or "+" and the slurry characteristics were thus excellent. Moreover, based on the results for Examples 1-1, 1-2, and 1-9, which all had about the same zeta potentials, it was shown that Examples 1-1 and 1-2, which had average fiber widths of less than or equal to 5 nm, presented even better slurry characteristics than did Example 1-9, which had an average fiber width for the nanocellulose of 5.3 nm.

In contrast to this, with Comparative Examples 1-1 to 1-4, the dispersion stability was evaluated as "×" and the slurry characteristics were also inferior to those of Examples 1-1 to 1-9. While Comparative Example 1-5 did have a good dispersion stability, all of its slurry characteristics were evaluated as "×".

[Second Example]

[Examples 2-1 to 2-9 and Comparative Examples 2-1 to 2-5] : Examination of the average fiber width

The same evaluations as in the First Example were carried out using the aqueous CNF dispersions A to H, J, and P to T respectively obtained in Production Examples 1 to 8 and 10 and Comparative Production Examples 1 to 5, and the relationship between the average fiber width of the nanocellulose and its dispersion stability was examined. The results of the evaluations are given in Table 2.

[Table 2]

| | designation of aqueous dispersion | N-oxyl compound | oxidation method | carboxy group content (mmol/g) | zeta potential (mV) | average fiber width (nm) | light transmittance (%) | stability of the aqueous CNF dispersion | | slurry viscosity stability | slurry handling characteristics | surface condition after coating with slurry |
|---|---|---|---|---|---|---|---|---|---|---|---|---|
| | | | | | | | | dispersion ratio (%) | rank | | | |
| Example 2-1 | A | × | hypochlorite | 0.37 | -30.2 | 4.7 | 95.5 | 94.2 | + | + | + | + |
| Example 2-2 | B | | | 0.45 | -32.5 | 4.5 | 97.8 | 98.9 | + + | + | + | + |
| Example 2-3 | C | | | 0.61 | -52.9 | 3.7 | 99.2 | 99.4 | + + | + + | + + | + + |
| Example 2-4 | D | | | 0.92 | -65.7 | 2.7 | 99.5 | 99.5 | + + | + + | + + | + + |
| Example 2-5 | E | | | 0.97 | -75.6 | 2.3 | 99.7 | 99.7 | + + | + | + + | + |
| Example 2-6 | F | | | 0.94 | -67.8 | 2.3 | 99.5 | 99.4 | + + | + + | + + | + + |
| Example 2-7 | G | | | 0.95 | -70.2 | 2.1 | 99.6 | 99.5 | + + | + | + + | + |
| Example 2-8 | H | | | 1.15 | -80.5 | 2.0 | 99.8 | 99.8 | + + | Δ | + + | Δ |
| Example 2-9 | J | | | 0.35 | -28.5 | 4.8 | 95.1 | 93.5 | + | Δ | + | Δ |
| Comparative Example 2-1 | P | × | hypochlorite | 0.42 | -17.9 | 5.3 | 84.7 | 83.3 | × | Δ | Δ | Δ |
| Comparative Example 2-2 | Q | | | 1.12 | -21.7 | 5.2 | 90.7 | 84.7 | × | Δ | Δ | Δ |
| Comparative Example 2-3 | R | | ozone oxidation | 0.43 | -33.6 | 5.5 | 78.6 | 83.8 | × | Δ | Δ | Δ |
| Comparative Example 2-4 | S | | periodic acid | 1.72 | -16.0 | 19.3 | 78.0 | 80.5 | × | × | × | × |
| Comparative Example 2-5 | T | + | TEMPO oxidation | 1.55 | -70.9 | 3.4 | 99.4 | 99.5 | + + | × | × | × |

EP 4 180 461 A1

19

According to Table 2, when Comparative Examples 2-1 and 2-2 are compared with Examples 2-1 to 2-9, in which examples the nanocellulose was produced by an oxidation treatment using hypochlorite, Examples 2-1 to 2-9, which had average fiber widths of not more than 5.0 nm, exhibited better slurry characteristics than for Comparative Examples 2-1 and 2-2, which had average fiber widths of 5.3 nm and 5.2 nm, respectively.

In addition, based on the results for Examples 2-1 and 2-9, which had about the same average fiber widths, it was shown that Example 2-1, which had a zeta potential of less than or equal to -30 mV, presented better slurry characteristics than did Example 2-9, which had a zeta potential for the nanocellulose of -28.5 mV.

[Third Example]

[Examples 3-1 to 3-9 and Comparative Examples 3-1 to 3-5] : Examination of the aspect ratio

Using the aqueous CNF dispersions A to H, K, and P to T obtained in the individual Production Examples 1 to 8 and 11 and Comparative Production Examples 1 to 5, the average fiber length and average fiber width of the nanocellulose were measured and the aspect ratio was calculated; the same evaluations as in the First Example were performed; and the relationship between the aspect ratio and dispersion stability for the nanocelluloses was examined. The average fiber length and average fiber width were measured using the following procedure.

[Measurement of the average fiber length and average fiber width]

Pure water was added to each of the aqueous CNF dispersions A to H, K, and P to T obtained as described above in order to adjust the nanocellulose concentration in the aqueous CNF dispersion to 5 ppm. After the concentration adjustment, the aqueous CNF dispersion was naturally dried on a mica substrate, and shape observation of the nanocellulose was performed in AC mode using an "MFP-3D Infinity" scanning probe microscope from Oxford · Asylum. For the average fiber length, the obtained image was binarized and analyzed using "imaged" image processing software. The number-average fiber length was determined for 100 of the fibers using fiber length = perimeter length ÷ 2. For the average fiber width, using the software provided with the "MFP-3D Infinity", the number-average fiber width [nm] was determined using cross-sectional height in the shape image = fiber width for at least 50 of the fibers. The aspect ratio was also calculated using (average fiber length/average fiber width).

The results of the evaluations are given in Table 3.

[Table 3]

| | designation of aqueous dispersion | N-oxyl compound | oxidation method | carboxy group content (mmol/g) | zeta potential (mV) | aspect ratio (length/diameter) | light transmittance (%) | stability of the aqueous CNF dispersion | | slurry viscosity stability | slurry handling characteristics | surface condition after coating with slurry |
|---|---|---|---|---|---|---|---|---|---|---|---|---|
| | | | | | | | | dispersion ratio (%) | rank | | | |
| Example 3-1 | A | ✕ | hypochlorite | 0.37 | -30.2 | 147 | 95.5 | 94.2 | + | + | + | + |
| Example 3-2 | B | | | 0.45 | -32.5 | 123 | 97.8 | 98.9 | + + | + | + | + |
| Example 3-3 | C | | | 0.61 | -52.9 | 96 | 99.2 | 99.4 | + + | + + | + + | + + |
| Example 3-4 | D | | | 0.92 | -65.7 | 48 | 99.5 | 99.5 | + + | + + | + + | + + |
| Example 3-5 | E | | | 0.97 | -75.6 | 42 | 99.7 | 99.7 | + + | + | + + | + |
| Example 3-6 | F | | | 0.94 | -67.8 | 46 | 99.5 | 99.4 | + + | + + | + + | + + |
| Example 3-7 | G | | | 0.95 | -70.2 | 32 | 99.6 | 99.5 | + + | + | + + | + |
| Example 3-8 | H | | | 1.15 | -80.5 | 30 | 99.8 | 99.8 | + + | △ | + + | △ |
| Example 3-9 | K | | | 0.35 | -28.6 | 125 | 98.1 | 64.8 | + | △ | + | △ |
| Comparative Example 3-1 | P | ✕ | hypochlorite | 0.42 | -17.9 | 183 | 84.7 | 83.3 | ✕ | △ | △ | △ |
| Comparative Example 3-2 | Q | | | 1.12 | -21.7 | 165 | 90.7 | 84.7 | ✕ | △ | △ | △ |
| Comparative Example 3-3 | R | | ozone oxidation | 0.43 | -33.6 | 170 | 78.6 | 83.8 | ✕ | △ | △ | △ |
| Comparative Example 3-4 | S | | periodic acid | 1.72 | -16.0 | 184 | 78.0 | 80.5 | ✕ | ✕ | ✕ | ✕ |
| Comparative Example 3-5 | T | + | TEMPO oxidation | 1.55 | -70.9 | 291 | 99.4 | 99.5 | + + | ✕ | ✕ | ✕ |

As shown in Table 3, when Comparative Examples 3-1 and 3-2 are compared with Examples 3-1 to 3-9, in which examples nanocellulose was produced by an oxidation treatment using a hypochlorite, Examples 3-1 to 3-9, in which the aspect ratio was less than or equal to 150, gave better slurry characteristics than in Comparative Examples 3-1 and 3-2, in which the aspect ratio was 183 and 165, respectively.

In addition, based on the results for Examples 3-2 and 3-9, which had about the same aspect ratio, it was shown that Example 3-2, which had a zeta potential of less than or equal to -30 mV, exhibited better slurry characteristics than did Example 3-9, for which the zeta potential of the nanocellulose was -28.6 mV.

## Claims

1. A nanocellulose that is an oxide of a cellulose raw material by a hypochlorous acid or a salt thereof and that has an average fiber width of from at least 1 nm to not more than 200 nm, wherein the nanocellulose

   substantially does not comprise a N-oxyl compound and
   has a zeta potential of less than or equal to -30 mV.

2. The nanocellulose according to claim 1, wherein the average fiber width is from at least 1 nm to not more than 5 nm.

3. The nanocellulose according to claim 1 or 2, wherein the nanocellulose has an aspect ratio of from at least 20 to not more than 150.

4. The nanocellulose according to any one of claims 1 to 3, wherein a mixture made by mixing the nanocellulose with water to give a solids concentration of 0.1 mass% has a light transmittance of at least 95%.

5. A nanocellulose that is an oxide of a cellulose raw material by a hypochlorous acid or a salt thereof, wherein the nanocellulose

   does not comprise a N-oxyl compound and
   has an average fiber width of from at least 1 nm to not more than 5 nm.

6. A nanocellulose that is an oxide of a cellulose raw material by a hypochlorous acid or a salt thereof and that has an average fiber width of from at least 1 nm to not more than 200 nm, wherein the nanocellulose

   does not comprise a N-oxyl compound and
   has an aspect ratio of from at least 20 to not more than 150.

7. A nanocellulose that is an oxide of a cellulose raw material by a hypochlorous acid or a salt thereof and that has an average fiber width of from at least 1 nm to not more than 200 nm, wherein the nanocellulose

   does not comprise a N-oxyl compound and
   has a light transmittance of at least 95% for a mixture thereof made by mixing with water to give a solids concentration of 0.1 mass%.

8. A nanocellulose dispersion in which the nanocellulose according to any one of claims 1 to 7 is dispersed in a dispersion medium.

**INTERNATIONAL SEARCH REPORT**

| International application No. |
| --- |
| PCT/JP2021/025937 |

A. CLASSIFICATION OF SUBJECT MATTER
Int.Cl. C08B15/04(2006.01)i
FI: C08B15/04

According to International Patent Classification (IPC) or to both national classification and IPC

B. FIELDS SEARCHED

Minimum documentation searched (classification system followed by classification symbols)
Int.Cl. C08B15/04

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X | WO 2020/027307 A1 (TOAGOSEI CO., LTD.) 06 February | 1-8 |
| Y | 2020 (2020-02-06), examples, reference examples | 1-4, 8 |
| X | WO 2018/230354 A1 (TOAGOSEI CO., LTD.) 20 December | 1-8 |
| Y | 2018 (2018-12-20), paragraph [0032], examples | 1-4, 8 |
| X | JP 2017-193814 A (KANTO DENKA KOGYO CO., LTD.) 26 | 1-8 |
| Y | October 2017 (2017-10-26), paragraph [0015], examples | 1-4, 8 |
| Y | JP 2018-199753 A (NIPPON PAPER INDUSTRIES CO., LTD.) 20 December 2018 (2018-12-20), paragraph [0014] | 1-4, 8 |
| P, X | WO 2020/184177 A1 (TOAGOSEI CO., LTD.) 17 September 2020 (2020-09-17), paragraphs [0071]-[0074], [0078] | 1-8 |

| ☐ Further documents are listed in the continuation of Box C. | ☒ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 12 August 2021 | 24 August 2021 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**
Information on patent family members

International application No.

PCT/JP2021/025937

```
WO 2020/027307 A1  06 February 2020    (Family: none)

WO 2018/230354 A1  20 December 2018    EP 3640264 A1
                                       paragraph [0047], examples
                                       CA 3060188 A
                                       CN 110520448 A

JP 2017-193814 A   26 October 2017     (Family: none)

JP 2018-199753 A   20 December 2018    (Family: none)

WO 2020/184177 A1  17 September 2020   (Family: none)
```

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018230354 A

- WO 2020027307 A

**Non-patent literature cited in the description**

- *Sustainable Chem. Eng.,* 2020, vol. 8 (48), 17800-17806